# FASCICULE DE BREVET EUROPEEN

(11) **EP 3 194 372 B1**
(45) Date de publication et mention de la délivrance du brevet: **25.12.2019**
(21) Numéro de dépôt: 15785158.5
(22) Date de dépôt: 18.09.2015
(51) Int. Cl.: C07D 233/64

(54) **PROCEDE D'OBTENTION DE LA L-HERCYNINE PURE**
VERFAHREN ZUR HERSTELLUNG VON REINEM L-HERCYNIN
METHOD FOR PRODUCING PURE L-HERCYNINE

(30) Priorité: 19.09.2014 FR 1458869
(43) Date de publication de la demande: 26.07.2017
(73) Titulaire: Tetrahedron, 75001 Paris (FR)
(72) Inventeur: ERDELMEIER, Irène, F-75001 Paris (FR)
(74) Mandataire: Novagraaf Technologies
(86) Numéro de dépôt international: PCT/FR2015/052502
(87) Numéro de publication internationale: WO 2016/042273

(56) Documents cités:
- VERNON N. REINHOLD ET AL: "Synthesis of .alpha.-N-methylated histidines", JOURNAL OF MEDICINAL CHEMISTRY, vol. 11, no. 2, 1968, pages 258-260, XP055158261, ISSN: 0022-2623, DOI: 10.1021/jm00308a014 cité dans la demande
- V. NARESH CHARY ET AL: "Characterization of amino acid-derived betaines by electrospray ionization tandem mass spectrometry", JOURNAL OF MASS SPECTROMETRY, vol. 47, no. 1, 26 janvier 2012 (2012-01-26), pages 79-88, XP055158369, ISSN: 1076-5174, DOI: 10.1002/jms.2029 cité dans la demande
- IRENE ERDELMEIER ET AL: "Cysteine as a sustainable sulfur reagent for the protecting-group-free synthesis of sulfur-containing amino acids: biomimetic synthesis of l-ergothioneine in water", GREEN CHEMISTRY, vol. 14, no. 8, 20 juin 2012 (2012-06-20), page 2256, XP055158342, ISSN: 1463-9262, DOI: 10.1039/c2gc35367a
- FOSTER A B: "Deuterium isotope effects in the metabolism of drugs and xenobiotics: implications for drug design", ADVANCES IN DRUG RESEARCH, ACADEMIC PRESS, LONDON, GB, vol. 14, 1985, pages 1-40, XP009086953, ISSN: 0065-2490
- CHEAH ET AL.: ANTIOX.REDOX SIGNAL, vol. 26, no. 5, 1 January 2017 (2017-01-01), pages 193-206,

## Description

La présente invention a pour objet un procédé d'obtention à l'échelle industrielle de la L-hercynine pure.

Cette invention se rapporte à un nouveau procédé d'obtention de la L-hercynine pure sans aucune étape de purification par chromatographie. Plus particulièrement, cette invention concerne un nouveau procédé industriel d'obtention de L-hercynine pure qui inclut une étape de purification par électrodialyse, ainsi qu'un nouveau procédé de préparation de la L-hercynine à partir de L-histidine.

### Etat de l'Art :

Les composés de type bétaïne (ammonium quaternaire) sont parmi les plus abondantes molécules organiques azotées dans le sol après les acides aminés protéogéniques et non-protéogéniques. Parmi ces bétaïnes, la glycine bétaine et l'hercynine en sont les principaux représentants.
La L-hercynine est le dérivé bétaïne de la L-histidine.

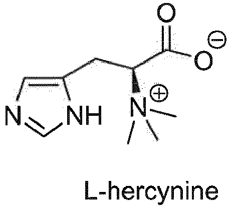

La L-hercynine est le précurseur direct de la L-ergothionéine (Y. Ishikawa et al., J. Biol. Chem.; 1974; 249; 4420 - 4427) chez certains champignons, mycobactéries et cyanobactéries (Pfeiffer, C. et al. ; Food Chemistry ; 2011 ; 129; 4 ; 1766-1769). Elle a donc été naturellement retrouvée chez ces microorganismes (Mahmood, Z.A. et al.; Pak. J. Pharm. Sci. ; 2010 ; 23 ; 349-357) mais aussi chez certains serpents (Ackermann, D. et al., ; Z. Physiol. Chem. Hoppe-Seyler's ; 1960 ; 318 ; 212-217). Très récemment, la L-hercynine a été mise en évidence, pour la première fois, dans des globules rouges humains (Chaleckis, R. et al.; Mol. Biosystems ; 2014 ; DOI 10.1039/c4mb00 346b).

La L-hercynine fait partie des substances régulatrices de la croissance des champignons tels que Agaricus bisporus (Champavier, Y. et al.; Enz. and Microbial. Technologt ; 2000 ; 26 ; 2-4 ; 243-251).

En tant que bétaïne, la L-hercynine pourrait jouer le rôle d'osmolyte, chez les plantes notamment (Velisek, J. ; Chemistry of Food ; J.Wiley & Sons Eds. ; 2013). En tant que tel, la L-hercynine exercerait une fonction cyto-protectrice en contribuant, notamment, au repliement correct des protéines dans les cellules.

Bien que ses caractéristiques biologiques soient considérables, la L-hercynine a été très peu étudiée sur le plan biologique; et ceci probablement en raison du fait qu'elle n'est pas disponible commercialement (Warren, C.R.; New Physiologist ; 2013 ; 198 ; 476-485).

Concernant sa préparation, que ce soit par voie directe (1 étape) ou indirecte (2 étapes), seuls 4 documents publiés à ce jour en font état, et ceci à l'échelle laboratoire.

En 1968, Melville et son équipe rapporte la première synthèse de L-hercynine à partir de L-histidine en 2 étapes (Reinhold, V. et al., ; J. Med. Chem.; 1968 ; 11 ; 258-260).

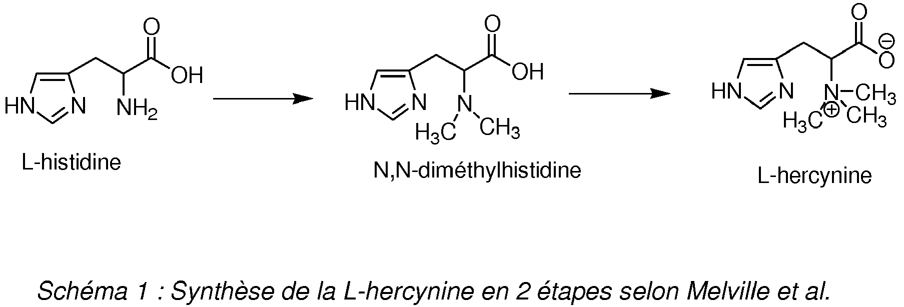

Au cours de la première étape, la L-histidine subit une méthylation en conditions réductrices à l'aide de formaldéhyde en présence de palladium sur charbon et d'hydrogène pour conduire à la L-N ,N'-di-méthyl-histidine. Après traitement à l'aide de solvants organiques, l'intermédiaire désiré est obtenu par recristallisation avec un rendement de 82%. La seconde étape consiste en une méthylation à l'aide d'iodure de méthyle à pH 9. La L-hercynine est obtenue après traitement et purification sur colonne échangeuse d'ions puis recristallisation avec un rendement de 89%. Il faut noter que les conditions utilisées par Melville ne sont pas racémisantes. Le rendement global de synthèse de la L-hercynine pure à partir de L-histidine selon cette procédure s'élève donc à 73%.

Ayant testé la voie directe de triméthylation de la L-histidine, Melville fait état de *«difficultés dans les essais de préparer la L-hercynine par méthylation directe de L-histidine notamment en raison de la méthylation concomitante du noyau imidazole qui se produit dans les conditions utilisées et du dérivé pentaméthylé correspondant qui se forme au cours de la réaction»* (Reinhold, V. et al.; J. Med. Chem.; 1968 ; 11 ; 260).

Plus récemment, un second document mentionne l'hercynine en tant qu'intermédiaire non isolé dans la préparation de l'acide urocanique, objectif désiré des auteurs de cette publication (Valeev, F.A. et al., Chemistry of Natural Compounds; 2007; 43(2);143-148) selon :

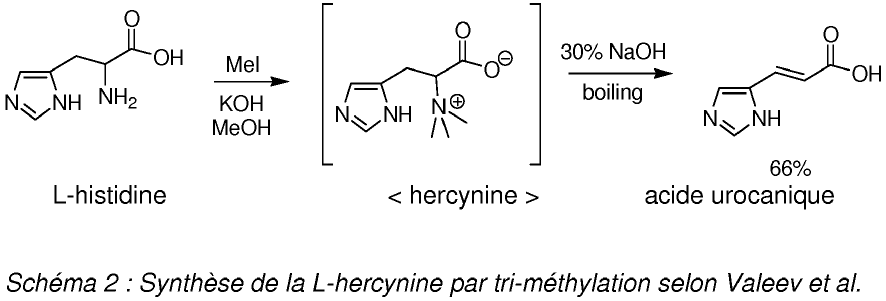

Les auteurs évoquent la méthylation « one-step » de la L-histidine pour obtenir intermédiairement l'hercynine qui, en milieu fortement basique et à chaud, conduit, après élimination de la triméthylamine, à l'acide urocanique désiré avec un rendement global de 66% à partir de la L-histidine. N'ayant pas isolé l'hercynine intermédiaire, ni mis en évidence la triméthylamine comme sous-produit, la formation de cette dernière n'est qu'hypothétique. De plus, à aucun moment, ils ne font état de sous-produits provenant de la méthylation du noyau imidazole comme l'évoquait Melville dans sa publication en 1968. Enfin, comme le centre chiral est détruit au cours de la séquence réactionnelle aboutissant à l'acide urocanique, aucune information n'est disponible afin de déterminer si cette triméthylation directe est réalisée avec ou sans racémisation.

En 2012, la préparation (en solution et en mélange avec les produits de per-méthylation) de l'hercynine par méthylation directe pour des études analytiques a été réalisée en utilisant un très large excès (16 équivalents) d'agent méthylant (Chary V. N. et al., J. Mass. Spectrom. 2012; 47 ; 79-88).

Enfin Khonde décrit, dans une thèse de Master en chimie, également la préparation de la L-hercynine par méthylation directe de la L-histidine, sans toutefois purifier la L-hercynine désirée (Khonde, L.P. ; Synthesis of Mycobacterial Ergothioneine biosynthetic pathway metabolites ; University of Cape Town, South Africa ; 2013 ; page 61, schéma 3.19).

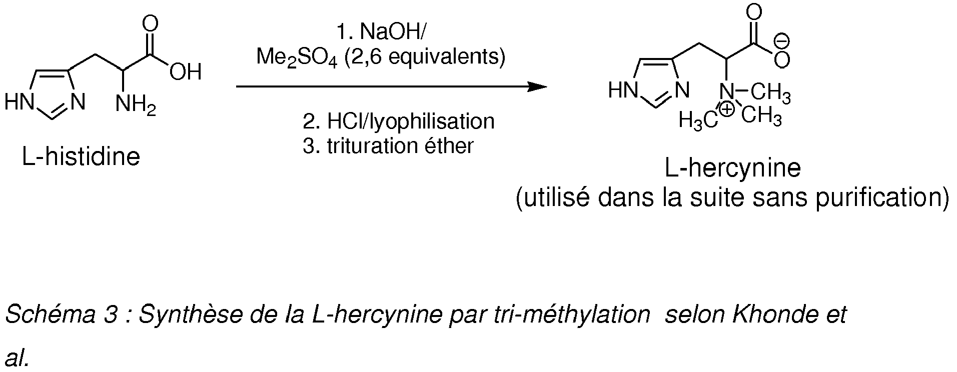

Khonde utilise le sulfate de diméthyle, comme agent méthylant, en milieu basique (NaOH 10%). Curieusement, seulement 2,6 équivalents de sulfate de diméthyle sont utilisés alors qu'au minimum 3 équivalents sont nécessaires théoriquement pour obtenir la L-hercynine à partir de la L-histidine. Par ailleurs, aucune donnée structurale n'est fournie par l'auteur qui utilise le mélange réactionnel brut pour l'étape suivante.

Alors qu'il existe plusieurs méthodes de synthèse chimiques de la L-hercynine en solution et en mélange avec des produits de perméthylation, comme nous l'avons vu ci-dessus, l'obtention de la L-hercynine pure est sérieusement limitée par les difficultés rencontrées lors de sa purification. En effet, la technique de purification la plus utilisée des acides aminés, bien connue de l'homme de l'art, à savoir la recristallisation s'avère très peu efficace voire totalement inutilisable en présence de quantité significative de sels. Seules des méthodes de purification par chromatographie d'échanges d'ions permettent d'obtenir la L-hercynine avec une bonne pureté. Dans le cadre de la préparation en 2 étapes décrite par Melville et déjà cité dans ce document (Reinhold, V. et al., ; J. Med. Chem.; 1968 ; 11 ; 258-260), pour obtenir un kilogramme de L-hercynine pure il faudrait utiliser 13,2 kg de résines échangeuses d'ions. A l'échelle industrielle cela représenterait des quantités considérables de résines qui doivent être éliminées dans le respect de la réglementation environnementale. Ce qui souligne l'importance de pouvoir disposer d'un procédé de purification qui évite l'utilisation de très grands volumes de résines.

REINHOLD et al, dans Journal of Medicinal Chemistry vol 11, N° 2, 1 mars 1968 pages 258-260, décrit aussi un procédé de préparation de l'Hercynine à partir de alpha-N,N-diméthyl-L-histidine.HCl.H2O dans le méthanol ajusté à 9, par addition de NH4OH, et réaction avec Mel à la température ambiante toute la nuit.

Le solvant est ensuite évaporé sous pression réduite pour donner un résidu solide blanc qui est ensuite traité sur colonne échangeuse d'ions en étant redissous dans un minimum d'eau.

Par ailleurs, ERDELMEIER et al dans Green Chemistry, 2012, 14, pages 2256-2265 décrit un procédé de désalage du milieu réactionnel contenant l'ergothionéine par électrodialyse. ERDELMEIER souligne que l'électrodialyse présente des avantages par rapport à la colonne échangeuse d'ions et conduit à une pureté élevée de l'ergothionéine.

Cependant, il n'était pas évident de remplacer le procédé de purification par colonne échangeuse d'ions de REINHOLD par le procédé d'électrodialyse utilisé par ERDELMEIER, même si l'ergothionéine et l'hercynine ont des structures voisines.

En effet, tout d'abord, le document ERDELMEIER mentionne l'utilisation de l'électrodialyse, mais souligne que cela est réalisé après l'étape de précipitation de la cystéine (« cysteine scavenging ») (voir page 2259, colonne de droite, 2ème §).

Or, il est bien précisé auparavant à la même page 2259, colonne de gauche que la précipitation de la cystéine se produit après ajustement du pH à 4-5.

Dans le cadre des premiers essais réalisés par les inventeurs avec l'électrodialyse, à un pH initial d'environ 7, des pertes de 18-25% de l'hercynine sont constatées, voir exemples comparatifs 1 et 2 en fin de la présente description.

Par contre, de manière surprenante, il a été découvert dans le cadre de la présente invention que si le pH est d'au moins 8, en, particulier environ 9, les pertes en hercynine sont nettement inférieures à 5%.

Cette chute inattendue des pertes en hercynine n'était pas du tout évidente pour un homme de l'art.

Il est encore à souligner que dans le document ERDELMEIER on ne dit rien au sujet du pH au démarrage de l'électrodialyse et surtout on ne parle pas de sa pertinence au niveau des pertes.

Il est encore à noter que le document ERDELMEIER mentionne un rendement global de 40% en ergothionéine sur les 2 étapes (voir page 2259, colonne de droite, 2E§.)

ERDELMEIER démontre ainsi que si l'électrodialyse aboutit à une pureté très élevée, le rendement en produit, à savoir l'ergothionéine, est obtenu avec un rendement global de 40%, démontrant à un homme de l'art des pertes importantes en ergothionéine, inacceptables à l'échelle industrielle.

Dans ces conditions, il n'était pas évident pour un homme de l'art de trouver des conditions pour l'hercynine avec des pertes < 5%, comme revendiqué dans le cadre de la présente invention.

Bien que son rôle physiologique soit encore mal défini, la L-hercynine est une molécule naturelle dont l'importance semble évidente. Il est donc essentiel de pouvoir disposer d'un procédé de préparation efficace de la L-hercynine pure aisément industrialisable, économiquement viable, non-racémisant et respectueux de l'environnement.

Sachant que la recristallisation s'avère très peu efficace voire totalement inutilisable en présence de quantité significative de sels, plusieurs difficultés doivent être solutionnées au-delà du problème relatif à la stéréochimie du produit final, à savoir:
1. la séparation complète de la L-hercynine, molécule très hydrophile, et des sels inorganiques, sous-produits de la réaction de méthylation (directe ou indirecte) par une méthode économiquement viable et industrialisable, excluant la chromatographie d'échange d'ions ;
2. une mise en œuvre limitant des pertes opérationnelles du produit désiré, c.à.d. < 5% de perte en L-hercynine au cours de l'étape de dessalage, et enfin
3. l'obtention de L-hercynine pure à partir d'un mélange contenant des dérivés structuralement très proches tels que la L-N-mono-méthyl-histidine, la L-N,N-di-méthyl-histidine, ou les produits provenant de la perméthylation comme évoqué par Melville.

### Buts de l'invention :

L'un des buts de la présente invention est de proposer un procédé simple et reproductible d'obtention de la L-hercynine pure, ou de son énantiomère la D-hercynine ou de son mélange racémique, ou de l'un de ses dérivés marqués isotopiquement incluant notamment une étape de purification permettant de séparer aisément la L-hercynine des sels inorganiques formés, préalablement à l'élimination des sous-produits organiques;
Ces buts sont atteints grâce à la présente invention qui repose sur un procédé de séparation de la L-hercynine des sels inorganiques, présents dans le milieu réactionnel, par électrodialyse; et cela quel que soit le mode de méthylation utilisé de la L-histidine ou de l'un de ses dérivés méthylés, suivie d'une purification par recristallisation. Ceci a été exemplifié dans la présente invention.

### Description de l'invention :

La présente invention a donc pour but de résoudre le nouveau problème technique consistant en la fourniture de la L-hercynine pure, ou de son énantiomère la D-hercynine ou de son mélange racémique, ou de l'un de ses dérivés marqués isotopiquement selon une solution qui englobe une étape de purification de la L-hercynine autre que la chromatographie d'échanges d'ions;
La Demanderesse a mis au point un nouveau procédé d'obtention de L-hercynine pure qui répond à tous ces critères, résolvant pour la première fois d'une manière simultanée tous les problèmes techniques énoncés, de façon très aisée et présentant de nombreux avantages sur les procédés existants. En particulier l'invention permet de fabriquer des grandes quantités permettant une utilisation du procédé à l'échelle industrielle. Par échelle industrielle, il est entendu une échelle ≥ 1kg, en particulier supérieure à 5Kg, mieux supérieure à 10Kg.

Ainsi dans son premier aspect, la présente invention concerne un procédé de purification de la L-hercynine, ou de son énantiomère D-hercynine ou de son mélange racémique, ou de l'un de ses dérivés marqués isotopiquement à partir d'un mélange réactionnel résultant de la réaction de la L-histidine, ou de son énantiomère D-histidine ou de son mélange racémique, ou de l'un de ses dérivés marqués isotopiquement ou de l'un de ses dérivés α-N-méthylés, dans des conditions de pH contrôlées, avec un agent de méthylation MeX dans un solvant polaire ou un mélange de solvants polaires, à température ambiante, caractérisé en qu'il comprend au moins une étape de séparation des produits organiques des sels inorganiques formés au cours de la réaction par électrodialyse ; et en ce que l'électrodialyse est conduite sur un milieu réactionnel ajusté à un pH compris entre 8.5 et 9.5, au démarrage de l'électrodialyse.

Selon un mode de réalisation particulier, l'invention concerne un procédé de purification caractérisé en ce qu'il comprend une étape supplémentaire de recristallisation de la L-hercynine , ou de son énantiomère D-hercynine ou de son mélange racémique, ou de l'un de ses dérivés marqués isotopiquement ou de l'un de ses dérivés méthylés, pour éliminer les impuretés organiques.

Selon un deuxième aspect, l'invention concerne un procédé de préparation à l'échelle industrielle de la L-hercynine, ou de son énantiomère D-hercynine ou de son mélange racémique, ou de l'un de ses dérivés marqués isotopiquement, ou de l'un de ses dérivés α-N-méthylés, caractérisé en qu'il comprend les étapes de :
▪ réaction de la L-histidine, ou de son énantiomère D-histidine ou de son mélange racémique, ou de l'un de ses dérivés marqués isotopiquement ou de l'un de ses dérivés α-N-méthylés, dans des conditions de pH contrôlées, avec un agent de méthylation MeX dans un solvant polaire ou un mélange de solvants polaires, à température ambiante; suivie de
▪ la séparation des produits organiques des sels inorganiques formés au cours de la réaction par électrodialyse et en ce que l'électrodialyse est conduite sur un milieu réactionnel ajusté à un pH compris entre 8.5 et 9.5, au démarrage de l'électrodialyse ; suivie de
▪ la recristallisation de la L-hercynine pour éliminer les impuretés organiques.

Par dérivés α-N-méthylés de la L-histidine, il est entendu la L-N-monométhyl-histidine ou la L-N,N'diméthyhistidine ou l'un de leurs sels.

Par température ambiante, on entend une température comprise entre 20°C et 35°C.

Pour chaque aspect de l'invention, on peut mettre en œuvre les modes de réalisation particuliers suivants :
Selon une mise en œuvre particulière, le procédé selon l'invention est caractérisé en ce que l'électrodialyse est conduite sur un milieu réactionnel ajusté à environ 9, au démarrage de l'électrodialyse. L'ajustement du pH peut être réalisé de manière classique par addition d'une base inorganique telle que la soude, la potasse ou la lithine.

Il a en effet été découvert de manière surprenante que les pertes en hercynine au cours de l'électrodialyse peuvent être réduites à < 5% si le pH du milieu aqueux est ajusté à 8,5 à 9,5 au démarrage de l'électrodialyse alors qu'en dehors de ce domaine, par exemple à environ 7, des pertes de 18-25% de l'hercynine sont constatées.

L'électrodialyse selon l'invention permet aussi d'aboutir à un désalage complet. Par désalage complet, on entend la séparation des sels du produit organique en solution aqueuse jusqu'à obtenir une proportion en sels inférieure à 1% en poids, ce qui correspond généralement à une conductivité finale de < 200µS/cm.

Il est tout aussi surprenant pour un homme de l'art que la méthode de l'électrodialyse soit applicable à la séparation complète des sels inorganiques/minéraux obtenus lors de l'étape de purification ou de préparation de la L-hercynine, en tant que bétaine, ou de son énantiomère D-hercynine ou de son mélange racémique, ou de l'un de ses dérivés marqués isotopiquement et ceci sans perte du produit significative (< 5%). Si l'électrodialyse est connue par l'homme de l'art en tant que méthode performante et très économique de dé-salage, des pertes significatives par migration transmembranaire sont cependant observées pour certains composés organiques, tels que des acides aminés (Eliseeva T. V. et al. , Desalination ; 2002 ; 149 ; 405-409, Sato et al., J. Membrane Sci. ; 1995 ; 100 ; 209-216) et en particulier pour des bétaïnes (EP 2216088 ; WO 9808803).

D'une manière générale, ces pertes, causées par diffusion, électromigration facilitée ou pression osmotique, sont d'autant plus importantes, si un dé-salage complet (< 1% de sels) est recherché, caractérisé par des valeurs faibles de conductivité finale (< 200µS/cm) et de courant limite (Shaposhnik V. A. et al., J. Membrane Sci. 1999 ; 161 ; 223-228). Pour le désalage industriel des bétaïnes tel que la 4-butyrobétaine par électrodialyse, il a même été proposé de combiner l'électrodialyse avec un retraitement des effluents afin de récupérer la bétaine perdue (EP2216088).

Selon une autre mise en œuvre particulière, le procédé selon l'invention est caractérisé en ce que l'agent méthylant MeX est choisi parmi les halogénures de méthyle, le sulfate de méthyle, le carbonate de méthyle ou encore le sulfonate de méthyle ou de trifluorométhyle ou de tosyle.

Selon encore une autre mise en œuvre particulière, le procédé selon l'invention est caractérisé en ce que le solvant polaire ou le mélange de solvants polaires est choisi parmi l'eau; un alcool en C1-C6 par exemple méthanol, éthanol, propanol ou propanol-2, butanol; l'acétate d'éthyle. Un mélange polaire particulier est un mélange eau/alcool, eau/propanol ou propanol-2, méthanol/acétate d'éthyle.

Selon une autre mise en œuvre particulière du procédé selon l'invention, les conditions de pH contrôlées sont choisies pour aboutir directement à une triméthylation de la L-histidine ou de son énantiomère D-histidine ou de son mélange racémique, ou de l'un de ses dérivés marqués isotopiquement ou de l'un de ses dérivés α-N-méthylés, ou indirectement à partir du dérivé diméthylé.

Selon une autre mise en œuvre particulière du procédé selon l'invention, les conditions de pH contrôlées sont choisies dans l'intervalle de pH = 9-13 par addition d'une base inorganique telle que la soude, la potasse ou la lithine. Il est important de noter qu'un pH plus faible, par exemple pH = 8,5, doit être évité sous peine d'une sélectivité nettement inférieure de la réaction ainsi que d'un rendement élevé en hercynine perméthylée ≥ 20% ;

Selon une autre mise en œuvre particulière du procédé selon l'invention, le mélange de solvants polaires pourrait être un mélange méthanol/eau.

Selon une autre mise en œuvre avantageuse du procédé selon l'invention, l'électrodialyse est réalisée en utilisant différentes membranes telles que des membranes anion-selectives (par exemple PC SA) et cation-selectives (par exemple PC MV) disponibles notamment chez PC Cell (Allemagne).

Les réactions subséquentes permettant de purifier et de recristalliser la L-hercynine pour la séparer des impuretés organiques, à partir du filtrat d'électrodialyse, sont effectuées dans des conditions classiques, connues de l'homme du métier.

Selon une autre variante de réalisation le procédé selon l'invention est caractérisé en ce qu'il peut être préparé la L-hercynine-d9 à partir d'un mélange réactionnel résultant de la réaction de la L-histidine avec un agent de méthylation tri-deutéré (d3), tel que l'iodométhane-d3.

Selon un troisième aspect, le procédé de l'invention fournit un composé L-hercynine-d9 pure, de formule

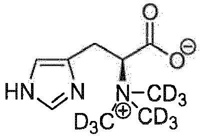

Ce procédé étant défini dans la description précédente ou la description suivante incluant les exemples.

Il est connu par le document Foster dans « Advances in Drug Research, Academic Press, London, GB, Vol 14, P 1-40, répertorié sous le N° XP009086953, ISSN 0065-2490» la deutération de composés pharmaceutiques. Cependant, Foster montre uniquement que les médicaments deutérés permettent d'identifier les métabolites lors de la biodégradation des molécules.

Dans le cadre de la présente invention, il est préparé spécifiquement et pour la première fois l'Hercynine-D9 pour laquelle les atomes D sont uniquement sur la partie liée à l'atome d'azote en position alpha. La préparation sélective en une étape par triméthylation avec le iodométhane d3 suivi par électrodialyse permet d'obtenir le produit d'une manière aisée, économique et en meilleur rendement, comparé avec la préparation alternative en 2 étapes. Cette préparation en 2 étapes serait évidente pour l'homme de l'art en analogie avec la préparation de l'hercynine non-deuteré selon le protocole de REINHOLD et al., dans Journal of Medicinal Chemistry vol. 11, N°2, 1 mars 1968 pages 258-260. Mais cette préparation en 2 étapes n'est économiquement pas viable parce qu'elle nécessite l'utilisation de trois réactifs contenant le deutérium : 1. il faudrait passer par amination réductrice avec le formaldehyde-D2, utilisant le deutérium gazeux D2 suivi par une avec le formaldehyde-D2, utilisant le deutérium gazeux D2 suivi par une quaternisation avec le iodométhane-D3. L'Hercynine-D9 a en outre ses propriétés intrinsèques.

Le procédé selon la présente invention selon chacun de ses aspects, présente l'avantage de ne pas faire appel à des réactifs dangereux tels que l'hydrogène, ni à l'utilisation de quantités importantes de résines échangeuses d'ions, comme dans le procédé de l'art antérieur (Reinhold, V. et al. ; J. Med. Chem.; 1968 ; 11 ; 258-260). Il est de plus réalisable à grande échelle, dans des solvants non toxiques comme par exemple l'eau ou un mélange eau/alcool. Outre l'aspect faisabilité, ces avantages réduisent considérablement non seulement les coûts de mise en œuvre mais aussi les impacts négatifs sur l'environnement.

Les problèmes techniques énoncés ci-dessus sont résolus pour la première fois d'une manière simultanée par la présente invention, de façon très aisée et économique, le procédé de préparation desdits nouveaux composés étant très simple à mettre en œuvre tout en fournissant de bons rendements.

### Description des figures :

**Figure 1** : Chromatogramme (HPLC) de la L-hercynine obtenu dans l'exemple 2 à l'echelle industrielle après dé-salage et recristallisation (détection universelle ELSD)
**Figure 2** : Chromatogramme (HPLC) de la L-hercynine-d9 obtenu dans l'exemple 3 après dé-salage et recristallisation (détection universelle ELSD)

### Exemples

Les exemples ci-après, de même que les figures, sont fournis simplement à titre d'illustration et ne sauraient, en aucune façon, limiter la portée de l'invention mais font cependant partie intégrante de l'invention, dans leurs moyens généraux.

Dans les exemples, décrits ci-après, tous les pourcentages sont donnés en poids, la température est la température ambiante ou est donnée en degré Celsius, et la pression est la pression atmosphérique, sauf indication contraire. Par température ambiante, on entend une température comprise entre 20 ° Cet 35 °C.

Les réactifs utilisés sont disponibles commercialement chez des fournisseurs internationaux tel que Sigma-Aldrich France (France), Alfa Aesar , Fisher Scientific, TCI Europe, Bachem (Suisse) sauf les composés suivants, qui ont été préparés selon le protocole cité : N,N-Diméthyl-L-histidine chlorohydrate hydrate (Reinhold, V. et al. ; J. Med. Chem.; 1968 ; 11 ; 258-260).

Le dé-salage a été réalisé par electrodialyse en utilisant des cellules constituées en alternance de membranes anion-selectives (par exemple PC SA) et cation-selectives (par exemple PC MV) disponibles notamment chez PC Cell (Allemagne), en utilisant des unités de desalination telles que l'unité ED 8002-001 (micro-unité pour l'echelle jusqu'à 10g), l'unité B-ED 1-3/ED 200 (jusqu'à 200g) et l'unité P20 (échelle industrielle), disponibles également chez PC Cell (Allemagne).

Les analyses RMN-¹H ont été enregistrées à 400 MHz dans le D₂O ou un mélange D₂O/DCI, en utilisant le signal de HOD (4.79 ppm) comme référence interne. Les déplacements chimiques sont notés en ppm, et la multiplicité des signaux indiquée par des symboles suivants : s (singlet), d (doublet), t (triplet), q (quartet), et m (multiplet). Les constantes de couplages sont notées en hertz (Hz). Les analyses RMN-¹³C sont enregistrées à 75 MHz dans le D₂O. Les analyses de masse sont obtenues par ionisation chimique à pression atmosphérique (APCI-MS). Les points de fusion ont été mesurés avec un appareil de la société Stuart Scientific. Les analyses HPLC ont été effectuées sur un appareil Acquity (Waters), en utilisant une colonne C8 250x4,6 (5µm). La phase mobile utilisée est un mélange eau/acétonitrile (98 :2) en 12 min et un débit de 0,6 ml/min. La détection s'effectue avec un détecteur universel ELSD (Sedere).

### I. Exemples selon l'invention du procédé de préparation des composés purs

Les exemples suivants illustrent un nouveau procédé simple et reproductible d'obtention de la L-hercynine pure, ou de son énantiomère la D-hercynine ou de son mélange racémique, ou de l'un de ses dérivés marqués isotopiquement qui repose sur un procédé de séparation des sels inorganiques, présents dans le milieu réactionnel, par électrodialyse d'une solution aqueuse ajustée à pH = 9; et cela quel que soit le mode de méthylation utilisé de la L-histidine ou de l'un de ses dérivés méthylés, suivie d'une purification par recristallisation.

### Exemple 1 de l'invention : Isolation de la L-hercynine pure à partir d'un mélange réactionnel obtenu par triméthylation de la L-histidine

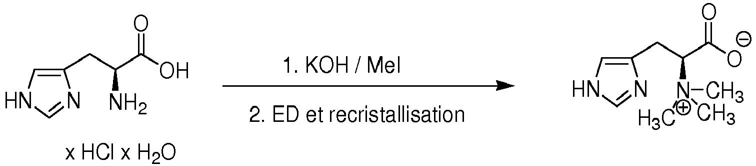

275g (1,3 Moles) du chlorhydrate hydrate de la L-histidine sont solubilisés dans 2,7 litres d'un mélange d'eau/méthanol (2 :1). Le pH est ajuste à 9-13 avec une solution d'hydroxyde de potassium 3M. Ensuite, 645g (4,55 Moles) d'iodométhane sont ajoutés sous agitation, et le mélange ainsi obtenu est agité pendant 18h à 25-35°C en maintenant le pH entre 9-13. Ensuite, le mélange est neutralisé en ajoutant de l'acide chlorhydrique aqueux à 37%. Après évaporation du méthanol, le pH de la solution aqueuse, contenant le produit brut en mélange avec les sels de chlorure et d'iodure de potassium, est ajusté à 9 avec une solution de NaOH (30%), puis cette solution est désalée par électrodialyse jusqu'à obtenir une conductivité de 50-100µS. Un test de présence d'halogène avec le nitrate d'argent, effectué sur un échantillon prélevé, confirme l'absence d'ions chlorure et iodure.

La solution aqueuse, obtenue après électrodialyse, est évaporée à sec. Le produit brut est purifié par recristallisation dans l'isopropanol aqueux, et la L-hercynine est obtenue après filtration et séchage (192g ; 73%) sous forme d'une poudre blanche.
[α]D = +44,5° (c = 1,0 ; 5N HCl)
pF : 238°C (dec.)
RMN-¹H (D₂O, 400 MHz) : δ (ppm) = 3,24 (m+s, 11 H), 3,90 (dd, J = 10Hz, J = 5Hz, 1H), 6,98 (s, 1H), 7,69 (s, 1H).
RMN-¹H (D₂O/DCI, 400 MHz) : δ (ppm) = 3,31 (s, 9H), 3,44 (dd, J = 14Hz, J = 12Hz, 1H), 3,55 (dd, J = 14Hz, J = 4 Hz, 1H), 4,13 (dd, J = 12Hz, J = 4Hz, 1H), 7,37 (s, 1H), 8,66 (s, 1H).
RMN-¹³C (D₂O, 75 MHz) : δ (ppm) = 25,7; 52,4; 78,9; 116,7; 132,3; 136,5; 171,2.
HPLC-MS (AP+) : m/z = 198 (MH+)
Pureté par HPLC (détection ELSD): 100%

### Exemple 2 de l'invention: Isolation de la L-Hercynine pure à partir d'un mélange réactionnel obtenu par triméthylation de la L-histidine à l'échelle industrielle

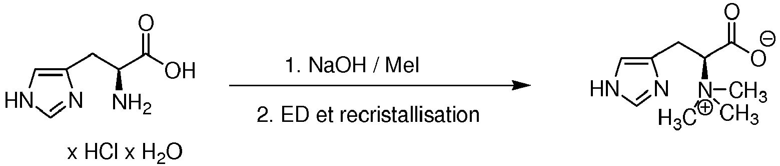

48,0 kg (229 Moles) du chlorhydrate hydrate de la L-histidine sont solubilisés dans 475 litres d'un mélange d'eau/méthanol (2 :1). Le pH est ajusté à 9-13 avec une solution d'hydroxyde de sodium à 10%. Ensuite, 113,8 kg (801 Moles) d'iodométhane sont ajoutés sous agitation, et le mélange ainsi obtenu est agité pendant 18h à 25-35°C en maintenant le pH entre 9-13. Ensuite, le mélange est neutralisé en ajoutant de l'acide chlorhydrique aqueux à 37%.

Après évaporation du méthanol, le pH de la solution aqueuse, contenant le produit brut en mélange avec les sels de chlorure et d'iodure de sodium, est ajusté à 9 avec une solution de NaOH (30%), puis cette solution est dé-salée par électrodialyse jusqu'à obtenir une conductivité de 50-100µS. Un test de présence d'halogène avec le nitrate d'argent, effectué sur un échantillon prélevé, confirme l'absence d'ions chlorure et iodure.

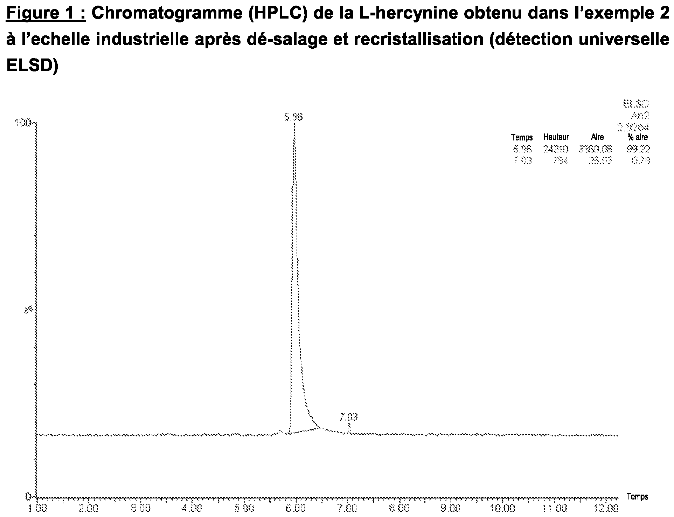

La solution aqueuse dé-salée, obtenue après électrodialyse, est concentrée. Après recristallisation dans l'isopropanol aqueux, la L-hercynine est obtenue après filtration et séchage (32,2 kg ; 72%) sous forme d'une poudre blanche.
[a]D : + 44° (c = 1,0 ; 5N HCl)
pF : 242°C (dec.)
Les spectres RMN-¹H et et de masse sont identiques à ceux de la L-hercynine obtenus dans l'exemple 1.
Pureté par HPLC (détection ELSD): 99,2% (voir Figure 1).

### Exemple 3 de l'invention: Isolation de la L-Hercynine-d9 pure à partir d'un mélange réactionnel obtenu par triméthylation de la L-histidine

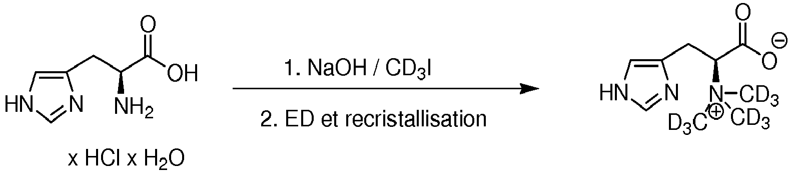

31,4g (150mmoles) du chlorhydrate hydrate de la L-histidine sont solubilisés dans 300 mL d'un mélange d'eau/méthanol (2 :1). Le pH est ajusté à 9-13 avec une solution d'hydroxyde de sodium 3M. Ensuite, 86,9g (600mmoles) d'iodométhane-d3 sont ajoutés progressivement sous agitation, en maintenant le pH entre 9-13. Après 18h d'agitation à 30°C, le mélange ainsi obtenu est neutralisé en ajoutant de l'acide chlorhydrique aqueux à 37%.

Après évaporation du méthanol, le pH de la solution aqueuse, contenant le produit brut en mélange avec les sels de chlorure et iodure de sodium, est ajusté à 9 avec une solution de NaOH (30%), puis cette solution est désalée par électrodialyse jusqu'à obtenir une conductivité de 50-100µS. Un test de présence d'halogène avec le nitrate d'argent, effectué sur un échantillon prélevé, confirme l'absence de ions chlorure et iodure.

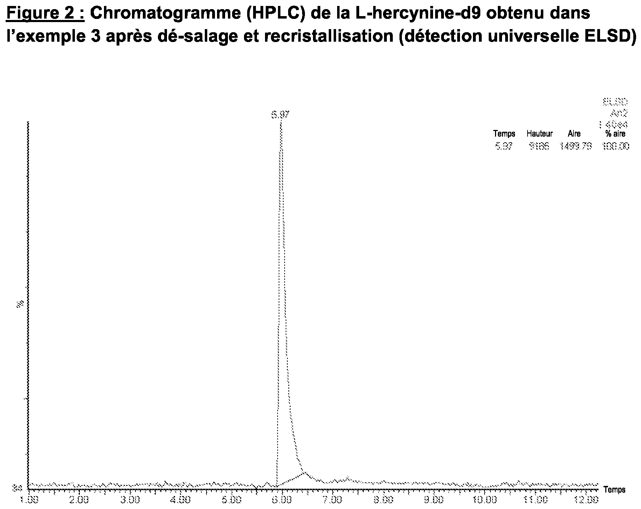

La solution aqueuse, obtenue après électrodialyse, est évaporée à sec. Le produit brut obtenu est purifié par recristallisation dans l'isopropanol aqueux, et la L-Hercynine-d9 est obtenue après filtration et séchage (23,8g ; 77%) sous forme d'une poudre blanche.
RMN-¹H (D₂O, 400 MHz) : δ (ppm) = 3,14 (m, 2H) ; 3.83 (dd, J = 10Hz, J = 5 Hz, 1 H) ; 6,92 (s, 1H) ; 7,62 (s, 1H).
HPLC-MS (AP+) : m/z = 207 (MH+).
Pureté par HPLC (détection ELSD): 100% (voir Figure 2).

### Exemple 4 de l'invention: Isolation de la L-Hercynine pure à partir d'un mélange réactionnel obtenu par quaternisation de la N,N-diméthyl-histidine avec l'iodométhane

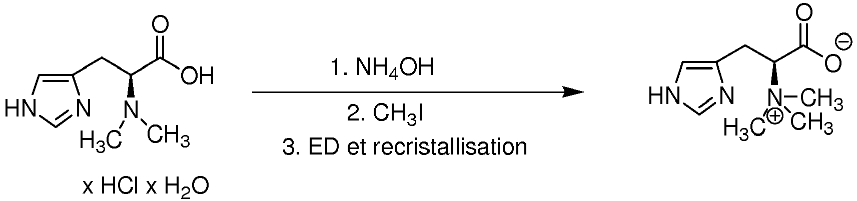

On procède pour la quaternisation de la N,N-diméthyl-histidine comme décrit par Reinhold, V. et al., ; J. Med. Chem.; 1968 ; 11 ; 258-260. Brièvement, 48,5g (200 mmoles) du chlorhydrate hydrate de la N,N-diméthyl-histidine sont solubilisés dans 1 litre de méthanol. Le pH est ajusté à 9 avec une solution aqueuse d'ammoniaque à 20%. Ensuite, 37,3g (16 mL, 1,3 équivalents) d'iodométhane sont ajoutés sous agitation. La solution ainsi obtenue est agitée pendant 18h à 30°C (température ambiante). Après évaporation du méthanol, le produit brut, dans le mélange réactionnel contenant les sels de chlorure et iodure d'ammonium (88g), est solubilisé dans 500mL d'eau, le pH est ajusté à 9 avec une solution de NaOH (30%), puis la solution est dé-salée par électrodialyse jusqu'à obtenir une conductivité de 43 micro-siemens (µS). Un test au nitrate d'argent, permettant d'estimer la présence d'ions halogénures, effectué sur un échantillon prélevé dans la solution aqueuse, confirme l'absence d'ions chlorure et iodure.

La solution aqueuse, obtenue après électrodialyse, est évaporée à sec. Le produit brut obtenu est purifié par recristallisation, et la L-hercynine est obtenue après filtration et séchage (36,6 g ; 93 %) sous forme d'une poudre blanche.
[α]D = +44° (c = 1,0 ; 5N HCl)
pF : 241 °C (dec.)
RMN-¹H (D₂O, 400 MHz) : δ (ppm) = 3,16 (m+s, 11H), 3,85 (dd, J = 10Hz, J = 5 Hz, 1H), 6,93 (s, 1H), 7,64 (s, 1H).
RMN-¹³C (D₂O, 75 MHz) : δ (ppm) = 25,7; 52,4; 78,9; 116,7; 132,3; 136,5; 171,2.
HPLC-MS (AP+) : m/z = 198 (MH+)

### Exemple 5 de l'invention: Isolation de la D-Hercynine pure à partir d'un mélange réactionnel obtenu par quaternisation de la D-N,N-diméthyl-histidine avec l'iodométhane

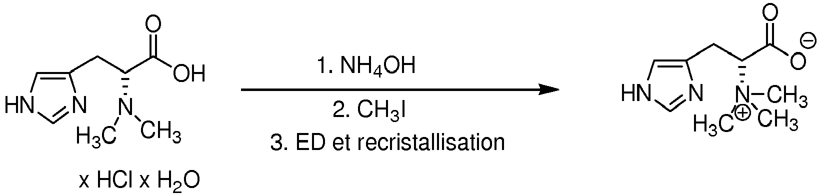

On procède pour la quaternisation de la D-N,N-diméthyl-histidine par analogie avec l'exemple 4 décrivant la synthèse et la purification de la L-hercynine. Brièvement, 21,3g (90mmoles) du chlorhydrate hydrate de la D-N,N-diméthyl-histidine sont solubilisés dans 0,45 litre de méthanol. Le pH est ajusté à 9 avec une solution aqueuse d'ammoniaque à 20%. Ensuite, 15,3g (6,7mL, 1,2 équivalents) d'iodométhane sont ajoutés sous agitation. La solution ainsi obtenue est agitée pendant 18h à 25-30°C. Après évaporation du méthanol, le produit brut, dans le mélange réactionnel contenant les sels de chlorure et iodure d'ammonium, est solubilisé dans 300mL d'eau. Après ajustement du pH de la solution à 9 avec une solution de NaOH (30%), le mélange est dé-salé par électrodialyse jusqu'à obtenir une conductivité de 70µS. Un test au nitrate d'argent, permettant d'estimer la présence d'ions halogénures, effectué sur un échantillon prélevé dans la solution aqueuse, confirme l'absence d'ions chlorure et iodure.

La solution aqueuse, obtenue après électrodialyse, est évaporée à sec. Le produit brut obtenu est purifié par recristallisation avec un mélange méthanol/acétate d'éthyle, et la L-hercynine est obtenue après filtration et séchage (16,7 g ; 93 %) sous forme d'une poudre blanche.
[a]D : - 44° (c = 1,0 ; 5N HCl)
Les spectres RMN-¹H et et de masse sont identiques à ceux de la L-hercynine obtenus dans l'exemple 1.

### Exemple 6 de l'invention: Isolation de la L-Hercynine pure à partir d'un mélange réactionnel obtenu par quaternisation de la N,N-diméthyl-histidine avec le sulfate de méthyle

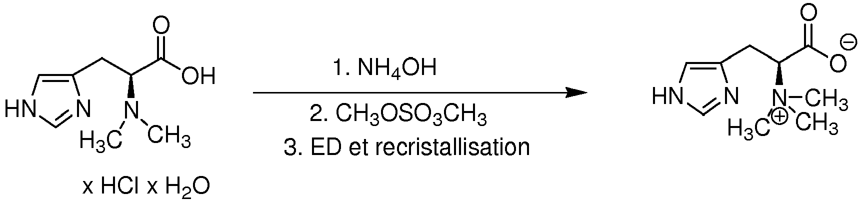

On procède pour la quaternisation de la N,N-diméthyl-histidine par analogie avec la procédure décrite par Reinhold, V. et al., ; J. Med. Chem.; 1968 ; 11 ; 258-260, en remplaçant l'iodométhane par le sulfate de méthyle. Brièvement 4,85g (20mmoles) du chlorhydrate hydrate de la N,N-diméthyl-histidine sont solubilisés dans 100mL de méthanol. Le pH est ajuste à 9 avec une solution aqueuse d'ammoniaque à 20%. Ensuite, 3,03g (2,28mL, 24mmoles, 1,2 équivalents) de sulfate de méthyle sont ajoutés sous agitation. La solution ainsi obtenue est agitée pendant 18h à 30°C. L'analyse par RMN-¹H d'un échantillon indique que la conversion n'est pas complète. 1,0g (0,75mL, 8mmoles, 0,4 équivalents) de sulfate de méthyle est ajouté au mélange réactionnel. Après agitation pendant 3h, le solvant est évaporé, et le produit brut, dans le mélange réactionnel contenant les sels de chlorure et iodure d'ammonium, est solubilisé dans 50mL d'eau. Après ajustement du pH à 9, un dé-salage par électrodialyse est réalisé jusqu'à obtenir une conductivité de 50µS, un test au nitrate d'argent, permettant d'estimer la présence d'ions halogénures, effectué sur un échantillon prélevé dans la solution aqueuse, ainsi qu'une analyse par HPLC-ELSD confirme l'absence de sels.

La solution aqueuse, obtenue après électrodialyse, est évaporée à sec. Après recristallisation du produit brut avec un mélange méthanol/acétate d'éthyle, la L-hercynine est obtenue après filtration et séchage (3,0 g ; 76 %) sous forme d'un poudre blanche.

Le spectre RMN-¹H et le pouvoir rotatoire sont identiques à ceux obtenus pour le produit isolé dans l'exemple 1.

### II. Exemples comparatifs du procédé d'électrodialyse

### Exemple comparatif 1: Evaluation de la séparation des sels inorganiques de la L-hercynine à pH 7 et pH 8

A) 2000 mL d'une solution aqueuse contenant 113g (0,57 mol) L-hercynine, 300g (3,5 équiv.) d'iodure de sodium et 33 g (1 équiv.) de chlorure de sodium (1,3 Moles) à pH 7 et une conductivité de 70 mS/cm sont placés dans le compartiment « diluât » d'un appareil d'électrodialyse, tel que par exemple l'unité B-ED 1-3/ED 200, équipé avec une cellule ED 200. 2000 mL d'eau déminéralisée sont placés dans le compartiment « concentrât ». 8000 mL d'une solution aqueuse de sulfate de sodium (0,5-1%) sont utilisés comme électrolyte. Le débit de circulation de toutes les solutions est de 100-120l/h, en appliquant une tension de 10-15V, jusqu'à ce que la conductivité du « diluât » soit < 0,1mS/cm. Un test de présence d'halogène avec le nitrate d'argent, effectué sur un échantillon prélevé, confirme l'absence d'ions chlorure et iodure. Le « diluât » contient 86g de la L-hercynine, correspondant à une perte de 24%.
B) 2000 mL d'une solution aqueuse contenant 113g (0,57 mol) L-hercynine, 300g (3,5 équiv.) d'iodure de sodium et 33 g (1 équiv.) de chlorure de sodium (1,3 Moles) à pH 8 et une conductivité de 75 mS/cm est électro-dialysé (comme décrit en A) jusqu'à une conductivité du « diluât » de 0,02mS/cm. Un test de présence d'halogène avec le nitrate d'argent, effectué sur un échantillon prélevé, confirme l'absence d'ions chlorure et iodure. Le diluât contient 98,3g de la L-hercynine, correspondant à une perte de 13%.

### Exemple comparatif 2 : Evaluation de la séparation des sels inorganiques de la L-hercynine à pH 7 (comparatif) et pH 9 (conforme à l'invention)

A) Le mélange réactionnel obtenu par triméthylation de la L-histidine, tel que décrit dans l'exemple 2 de l'invention, après neutralisation avec l'acide chlorhydrique HCl, contenant 5% (w/w) de l'hercynine en mélange avec des sous-produits de la réaction, ainsi que les sels de chlorure et d'iodure de sodium, est dé-salé en portion de 2000 mL par électrodialyse comme décrit dans l'exemple comparatif 1A, jusqu'à obtenir une conductivité de 50-100µS. Le contenu en L-hercynine est dosé par HPLC. La procédure a été appliquée sur 5 lots numérotés 1 à 5, et les pertes (allant de 22-25%) en L-hercynine sont répertoriées dans le tableau 1.
B) Dans les mêmes conditions, hormis le fait que le pH soit ajusté à 9, conformément à l'invention, avec l'hydroxyde de sodium à 30% dans le compartiment « diluât » au démarrage de l'électrodialyse, 5 lots numérotés 6 à 10 sont dé-salés jusqu'à obtenir une conductivité de 50-100µS. Le contenu en L-hercynine dans le « diluât » est dosé par HPLC. La procédure a été appliquée sur 5 lots, et les pertes en L-hercynine, allant de seulement 1,5 - 2,5%, sont répertoriées dans le Tableau 1.

| **Tableau 1: Pertes en L-hercynine** | | | | | | |
|---|---|---|---|---|---|---|
| Exemple 2A : pH = 7 | perte | Lot 1 | Lot 2 | Lot 3 | Lot 4 | Lot 5 |
| | | 24% | 22% | 25% | 22% | 23% |
| Exemple 2B : pH = 9 | perte | Lot 6 | Lot 7 | Lot 8 | Lot 9 | Lot 10 |
| | | 2,5% | 2,3% | 1,9% | 2% | 1,5% |

Les exemples comparatifs 1 et 2 montrent donc bien que les pertes au cours de l'électrodialyse peuvent être limitées d'une manière très significative en ajustant le pH de la solution aqueuse à 8,5-9,5, idéalement à environ 9, au démarrage de l'électrodialyse, conformément à l'invention.

## Revendications

1. Procédé de purification de la L-hercynine, ou de son énantiomère D-hercynine ou de son mélange racémique, ou de l'un de ses dérivés marqués isotopiquement à partir d'un mélange réactionnel résultant de la réaction de la L-histidine, ou de son énantiomère D-histidine ou de son mélange racémique, ou de l'un de ses dérivés marqués isotopiquement ou de l'un de ses dérivés α-N-méthylés, dans des conditions de pH contrôlées, avec un agent de méthylation MeX dans un solvant polaire ou un mélange de solvants polaires, à température ambiante, **caractérisé en ce qu'**il comprend au moins une étape de séparation des produits organiques des sels inorganiques formés au cours de la réaction par électrodialyse ; et **en ce que** l'électrodialyse est conduite sur un milieu réactionnel ajusté à un pH compris entre 8.5 et 9.5, au démarrage de l'electrodialyse .

2. Procédé de purification selon la revendication 1, **caractérisé en ce que** qu'il comprend une étape supplémentaire de recristallisation de la L-hercynine, ou de son énantiomère D-hercynine ou de son mélange racémique, ou de l'un de ses dérivés marqués isotopiquement ou de l'un de ses dérivés méthylés, pour éliminer les impuretés organiques.

3. Procédé de préparation à l'échelle industrielle de la L-hercynine, ou de son énantiomère D-hercynine ou de son mélange racémique, ou de l'un de ses dérivés marqués isotopiquement ou de l'un de ses dérivés α-N-méthylés, caractérisé en qu'il comprend les étapes de :
▪ réaction de la L-histidine, ou de son énantiomère D-histidine ou de son mélange racémique, ou de l'un de ses dérivés marqués isotopiquement ou de l'un de ses dérivés α-N-méthylés, dans des conditions de pH contrôlées, avec un agent de méthylation MeX dans un solvant polaire ou un mélange de solvants polaires, à température ambiante; suivie de
▪ la séparation des produits organiques des sels inorganiques formés au cours de la réaction par électrodialyse ; et en ce que l'électrodialyse est conduite sur un milieu réactionnel ajusté à un pH compris entre 8.5 et 9.5, au démarrage de l'electrodialyse.; suivie de
▪ la recristallisation de la L-hercynine pour éliminer les impuretés organiques.

4. Procédé selon l'une des revendications 1 à 3, **caractérisé en ce que** l'électrodialyse est conduite sur un milieu réactionnel ajusté à un pH à environ 9, au démarrage de l'electrodialyse.

5. Procédé selon l'une des revendications 1 à 4, **caractérisé en ce que** l'agent méthylant MeX est choisi parmi les halogénures de méthyle, le sulfate de méthyle, le carbonate de méthyle ou encore le sulfonate de méthyle ou de trifluorométhyle ou de tosyle.

6. Procédé selon l'une des revendications 1 à 5, **caractérisé en ce que** le solvant polaire ou le mélange de solvants polaires est choisi parmi l'eau; un alcool en C1-C6 par exemple méthanol, éthanol, propanol ou propanol-2, butanol; l'acétate d'éthyle; et un mélange eau/alcool, eau/propanol ou propanol-2 ; ou méthanol/acétate d'éthyle.

7. Procédé selon l'une des revendications 1 à 6, **caractérisé en ce que** les conditions de pH contrôlées sont choisies pour aboutir directement à une triméthylation de la L-histidine ou de son énantiomère D-histidine ou de son mélange racémique, ou de l'un de ses dérivés marqués isotopiquement ou de l'un de ses dérivés α-N-méthylés, ou indirectement à partir du dérivé diméthylé.

8. Procédé selon l'une des revendications 1 à 7, **caractérisé en ce que** les conditions de pH contrôlées sont choisies dans l'intervalle de pH = 9-13 par addition d'une base inorganique telle que la soude, la potasse ou la lithine.

9. Procédé selon l'une des revendications 1 à 8, **caractérisé en ce que** l'on prépare la L-Hercynine-d9 à partir d'un mélange réactionnel résultant de la réaction de la L-Histidine avec un agent de méthylation-d3, en particulier le iodométhane-d3, ladite L-Hercynine-d9, étant de formule

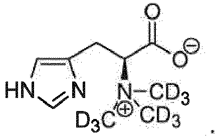

## Patentansprüche

1. Verfahren zur Reinigung von L-Hercynin oder seines Enantiomers, D-Hercynin, oder seines racemischen Gemisches oder eines seiner isotopenmarkierten Derivate ausgehend von einem Reaktionsgemisch, das aus der Reaktion von L-Histidin oder seines Enantiomers, D-Histidin, oder seines racemischen Gemisch oder eines seiner isotopenmarkierten Derivate oder eines seiner α-N-methylierten Derivate unter kontrollierten pH-Bedingungen mit einem Methylierungsmittel MeX in einem polaren Lösungsmittel oder einem Gemisch von polaren Lösungsmitteln bei Raumtemperatur resultiert, **dadurch gekennzeichnet, dass** es mindestens einen Schritt der Abtrennung organischer Produkte von während der Reaktion gebildeten anorganischen Salzen durch Elektrodialyse umfasst; und dass die Elektrodialyse mit einem Reaktionsmedium durchgeführt wird, das zu Beginn der Elektrodialyse auf einen pH-Wert zwischen 8,5 und 9,5 eingestellt wurde.

2. Reinigungsverfahren gemäß Anspruch 1, **dadurch gekennzeichnet, dass** es einen zusätzlichen Schritt der Umkristallisation des L-Hercynins oder seines Enantiomers D-Hercynin oder seines racemischen Gemischs oder eines seiner isotopenmarkierten Derivate oder eines seiner methylierten Derivate umfasst, um die organischen Verunreinigungen zu entfernen.

3. Verfahren zur großtechnischen Herstellung von L-Hercynin oder seines Enantiomers D-Hercynin oder seines racemischen Gemisches oder eines seiner isotopenmarkierten Derivate oder eines seiner α-N-methylierten Derivate, **dadurch gekennzeichnet, dass** es die folgenden Schritte umfasst:
• Reaktion von L-Histidin oder seines Enantiomers D-Histidin oder seines racemischen Gemisches oder eines seiner isotopenmarkierten Derivate oder eines seiner α-N-methylierten Derivate unter kontrollierten pH-Bedingungen, mit einem Methylierungsmittel MeX in einem polaren Lösungsmittel oder einem Gemisch von polaren Lösungsmitteln bei Raumtemperatur; gefolgt von
• Abtrennung organischer Produkte von während der Reaktion gebildeten anorganischen Salzen durch Elektrodialyse; wobei die Elektrodialyse mit einem Reaktionsmedium durchgeführt wird, das zu Beginn der Elektrodialyse auf einen pH-Wert zwischen 8,5 und 9,5 eingestellt wurde; gefolgt von
• Umkristallisation von L-Hercynin zum Entfernen der organischen Verunreinigungen.

4. Verfahren gemäß einem der Ansprüche 1 bis 3, **dadurch gekennzeichnet, dass** die Elektrodialyse mit einem Reaktionsmedium durchgeführt wird, das zu Beginn der Elektrodialyse auf einen pH-Wert von etwa 9 eingestellt wurde.

5. Verfahren gemäß einem der Ansprüche 1 bis 4, **dadurch gekennzeichnet, dass** das Methylierungsmittel MeX ausgewählt ist aus Methylhalogeniden, Methylsulfat, Methylcarbonat oder Methyl- oder Trifluormethyl- oder Tosylsulfonat.

6. Verfahren gemäß einem der Ansprüche 1 bis 5, **dadurch gekennzeichnet, dass** das polare Lösungsmittel oder das Gemisch aus polaren Lösungsmitteln ausgewählt ist aus Wasser; einem C1-C6-Alkohol, beispielsweise Methanol, Ethanol, Propanol oder 2-Propanol, Butanol; Ethylacetat; und einem Gemisch aus Wasser/Alkohol, Wasser/Propanol oder 2-Propanol; oder Methanol/Ethylacetat.

7. Verfahren gemäß einem der Ansprüche 1 bis 6, **dadurch gekennzeichnet, dass** die kontrollierten pH-Bedingungen so gewählt werden, dass sie zu einer Trimethylierung von L-Histidin oder seinem Enantiomer D-Histidin oder seinem racemischen Gemisch oder einem seiner isotopenmarkierten Derivate oder einem seiner α-N-methylierten Derivate direkt, oder indirekt ausgehend vom Dimethylderivat, führen.

8. Verfahren gemäß einem der Ansprüche 1 bis 7, **dadurch gekennzeichnet, dass** durch Zugeben einer anorganischen Base wie Natriumhydroxid, Kaliumhydroxid oder Lithiumhydroxid die kontrollierten pH-Bedingungen im Bereich von pH = 9-13 gewählt werden.

9. Verfahren gemäß einem der Ansprüche 1 bis 8, **dadurch gekennzeichnet, dass** das L-Hercynin-d9 ausgehend von einem Reaktionsgemisch hergestellt wird, das aus der Reaktion von L-Hisidin mit einem Methylierungsmittel-d3, insbesondere Iodmethan-d3, resultiert, wobei L-Hercynin-d9 die Formel

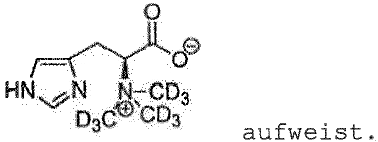

## Claims

1. A method for purifying L-hercynine, or its enantiomer, D-hercynine, or its racemic mixture, or one of its isotopically marked derivatives from a reaction mixture resulting from the reaction of L-histidine, or its enantiomer D-histidine or its racemic mixture, or one of its isotopically marked derivatives or one of its α-N-methylated derivatives, under controlled pH conditions, with a methylation agent MeX in a polar solvent or a mixture of polar solvents, at room temperature, **characterized in that** it comprises at least one step for separating the organic products from the inorganic salts formed during the reaction by electrodialysis; and **in that** the electrodialysis is conducted on a reaction medium adjusted to a pH comprised between 8.5 and 9.5, at the beginning of the electrodialysis.

2. The purification method according to claim 1, **characterized in that** it comprises an additional step for recrystallization of the L-hercynine, or its enantiomer, D-hercynine, or its racemic mixture, or one of its isotopically marked derivatives or one of its methylated derivatives, to eliminate the organic impurities.

3. A method for the industrial-scale production of L-hercynine, or its enantiomer, D-hercynine, or its racemic mixture, or one of its isotopically marked derivatives, or one of its α-N-methylated derivatives, **characterized in that** it comprises the following steps:
▪reacting the L-histidine, or its enantiomer D-histidine or its racemic mixture, or one of its isotopically marked derivatives or one of its α-N-methylated derivatives, under controlled pH conditions, with a methylation agent MeX in a polar solvent or a mixture of polar solvents, at room temperature; followed by
▪ separating the organic products from the inorganic salts formed during the reaction by electrodialysis; and **in that** the electrodialysis is done on a reaction medium adjusted to a pH comprised between 8.5 and 9.5, upon starting the electrodialysis; followed by
▪ re-crystallizing the L-hercynine to eliminate the organic impurities.

4. The method according to one of claims 1 to 3, **characterized in that** the electrodialysis is done on a reaction medium adjusted to about 9, at the beginning of the electrodialysis.

5. The method according to one of claims 1 to 4, **characterized in that** the methylating agent MeX is chosen from among methyl halogenide, methyl sulfate, methyl carbonate or methyl or trifluoromethyl or tosyl sulfonate.

6. The method according to one of claims 1 to 5, **characterized in that** the polar solvent or the mixture of polar solvents is chosen from among water; a C1-C6 alcohol, for example methanol, ethanol, propanol or propanol-2, butanol; ethyl acetate. One particular polar mixture is a water/alcohol, water/propanol or propanol-2, methanol/ethyl acetate mixture.

7. The method according to one of claims 1 to 6, **characterized in that** the controlled pH conditions are chosen to result directly in a tri-methylation of the L-histidine or its D-histidine enantiomer or its racemic mixture, or one of its isotopically marked derivatives or one of its α-N-methylated derivatives, or indirectly from the di-methylated derivative.

8. The method according to one of claims 1 to 7, **characterized in that** the controlled pH conditions are chosen in the interval pH = 9-13 by adding an inorganic base such as sodium hydroxide, potassium or lithium hydroxide.

9. The method according to one of claims 1 to 8, **characterized in that** the L-Hercynine-d9 is prepared from a reaction mixture resulting from the reaction of the L-Histidine with a methylation-d3 agent, in particular iodomethane-d3, said L-Hercynine-d9, being of formula:
